# EUROPEAN PATENT APPLICATION

(11) **EP 3 834 624 A1**
(43) Date of publication of application: **16.06.2021**
(21) Application number: 19846089.1
(22) Date of filing: 05.08.2019
(51) Int. Cl.: A23L 3/3499, A01N 43/16, A01P 3/00, A23C 9/152, A23C 11/10, A23F 3/16, A23F 5/24, A23L 2/00, A23L 2/02, A23L 2/38, A23L 2/44, A23L 2/52, A61K 31/12, A61K 36/185, A61P 19/00, A61P 31/04, A61P 35/00, A61P 39/06

(54) **AGENT FOR SUPPRESSING HEAT-RESISTANT ACIDOPHILIC BACTERIA, METHOD FOR PREVENTING CONTAMINATION WITH HEAT-RESISTANT ACIDOPHILIC BACTERIA, AND BEVERAGE**

(30) Priority: 10.08.2018 JP 2018151538
(71) Applicant: Suntory Holdings Limited, Osaka 530-8203 (JP)
(72) Inventor: FUKIZAWA, Shinya, Soraku-gun, Kyoto 619-0284 (JP); NONAKA, Yuji, Soraku-gun, Kyoto 619-0284 (JP); WAKABAYASHI, Kenichi, Soraku-gun, Kyoto 619-0284 (JP); YAMASHITA, Mai, Soraku-gun, Kyoto 619-0284 (JP)
(74) Representative: Vossius & Partner Patentanwälte Rechtsanwälte mbB
(86) International application number: PCT/JP2019/030716
(87) International publication number: WO 2020/031955

(57) **Abstract**

The present invention aims to provide a Thermo-Acidophilic Bacilli inhibitor, a method of preventing contamination by Thermo-Acidophilic Bacilli, and a beverage, which utilize as an active ingredient a substance having the effect of inhibiting the growth of Thermo-Acidophilic Bacilli. The present invention relates to a Thermo-Acidophilic Bacilli inhibitor containing xanthohumol as an active ingredient, a method of preventing contamination by Thermo-Acidophilic Bacilli using the same, and a beverage containing the same.

## Description

### TECHNICAL FIELD

The present invention relates to a Thermo-Acidophilic Bacilli inhibitor containing xanthohumol, a method of preventing contamination by Thermo-Acidophilic Bacilli using xanthohumol, and a beverage.

### BACKGROUND ART

Xanthohumol is a prenylflavonoid derived from hops and known to have various physiological activities such as a cancer cell growth inhibitory action, an antioxidant action, a bone degradation inhibitory action, and an antibacterial action.

In expectation of maintenance and improvement of health by these physiological activities of xanthohumol, attempts have been made to increase the amount of xanthohumol in beverages (Patent Literature 1 and Patent Literature 2). The amount of xanthohumol in non-fermented beverages, however, is typically very small.

Also, for example, Patent Literature 3 discloses a preservative for food containing xanthohumol of a hop cone component as an active ingredient, wherein xanthohumol has an antibacterial action against specific putrefactive bacteria (e.g., *Bacillus subtilis*). However, whether or not xanthohumol has an antibacterial action against other putrefactive bacteria is not known, and Patent Literature 3 does not mention the antibacterial action against Thermo-Acidophilic Bacilli.

It is important to prevent contamination of commercially available beverages by microorganisms such as yeast, bacteria, and mold and thereby increase the preservability of the beverages. Typical methods of preventing contamination by microorganisms include: decreasing the pH to a value in the acidic range; lowering the water activity by drying or the like; heating; storing at low temperatures; adding a preservative; aseptic packaging; and nitrogen flush packaging. In particular, the heating, for example for production of soft drinks, requires to be conducted under the sterile conditions in conformity with the Food Sanitation Act.

Even such a method of preventing contamination by microorganisms in the process of producing beverages still involves a risk where microorganisms that cause deterioration of quality survive and grow. For example, even after heating an acidic beverage, in which microorganisms tend not to grow naturally, under the sterile conditions in conformity with the Food Sanitation Act (the standards for food, including soft drinks), Thermo-Acidophilic Bacilli (TAB) from the raw materials may survive in the beverage. Thermo-Acidophilic Bacilli belong to the genus Alicyclobacillus and they are gram-positive, spore-forming bacteria. The representative species thereof is *Alicyclobacillus acidoterrestris.* Thermo-Acidophilic Bacilli do not die immediately in the typical thermal sterilization under the acidic conditions with a pH of 6.5 or lower. Thermo-Acidophilic Bacilli are known to deteriorate the quality of food and beverages when grown, with their action to convert ferulic acid in fruit juice to guaiacol which is an odor-causing substance. Known methods to prevent this include sterilization at higher temperatures than the standard temperature and use of benzoic acid. These methods, however, have not been put into practical use because the sterilization at higher temperatures than the standard temperatures spoil the flavors of beverages and benzoic acid is a synthetic preservative.

Hop extracts including xanthohumol have been used as preservatives for enhancement of the preservability of food other than beverages, owing to their antibacterial action against certain putrefactive bacteria. However, use of xanthohumol for prevention of contamination of beverages by microorganisms has not been considered. Also, use of xanthohumol has not been considered for prevention of contamination of acidic beverages, in which microorganisms tend not to grow, by Thermo-Acidophilic Bacilli that do not die immediately under the ordinary sterile conditions.

### CITATION LIST

### - Patent Literature

Patent Literature 1: JP 2002-345433 A
Patent Literature 2: JP 2003-310240 A
Patent Literature 3: JP 4374123 B

### SUMMARY OF INVENTION

### - Technical Problem

The present invention aims to provide a Thermo-Acidophilic Bacilli inhibitor, a method of preventing contamination by Thermo-Acidophilic Bacilli, and a beverage, which utilize as an active ingredient a substance having the effect of inhibiting the growth of Thermo-Acidophilic Bacilli.

### - Solution to Problem

As a result of intensive studies to solve the above problems, the present inventors discovered that xanthohumol inhibits the growth of Thermo-Acidophilic Bacilli and has an antibacterial action against Thermo-Acidophilic Bacilli. The present inventors also found applicability of xanthohumol to various beverages and the like because xanthohumol is a highly safe compound contained in edible plants. The present invention was thus completed.

Specifically, the present invention relates to the following Thermo-Acidophilic Bacilli inhibitor, method of preventing contamination by Thermo-Acidophilic Bacilli, and beverage.
[1] A Thermo-Acidophilic Bacilli inhibitor containing xanthohumol as an active ingredient.
[2] The Thermo-Acidophilic Bacilli inhibitor according to [1] above, which is for beverage use.
[3] The Thermo-Acidophilic Bacilli inhibitor according to [1] or [2] above, wherein Thermo-Acidophilic Bacilli are *Alicyclobacillus acidoterrestris.*
[4] A method of preventing contamination of a beverage by Thermo-Acidophilic Bacilli, including adding xanthohumol to a concentration of 3.1 mass ppm or more.
[5] The method of preventing contamination by Thermo-Acidophilic Bacilli according [4] above, wherein xanthohumol is added to a concentration of 6.2 mass ppm or more.
[6] The method of preventing contamination by Thermo-Acidophilic Bacilli according to [4] or [5] above, further including adjusting a pH of the beverage to 6.5 or lower.
[7] The method of preventing contamination by Thermo-Acidophilic Bacilli according to any one of [4] to [6] above, wherein the Thermo-Acidophilic Bacilli are *Alicyclobacillus acidoterrestris.*
[8] A beverage containing more than 12 mass ppm and 180 mass ppm or less of xanthohumol and having a pH of 6.5 or lower.
[9] The beverage according to [8] above, which contains 60 mass ppm or more of xanthohumol.
[10] The beverage according to [8] or [9] above, which is a tea-based beverage, coffee beverage, alcohol beverage, non-alcoholic beer taste beverage, carbonated beverage, functional beverage, fruit and/or vegetable-based beverage, lactic beverage, soy milk beverage, or flavored water.
[11] The beverage according to any one of [8] to [10] above, which is a non-alcoholic beer taste beverage, functional beverage, carbonated beverage, or fruit and/or vegetable-based beverage and has a pH of 4.6 or lower.

### - Advantageous Effects of Invention

The present invention can provide a Thermo-Acidophilic Bacilli inhibitor, a method of preventing contamination by Thermo-Acidophilic Bacilli, and a beverage, which utilize as an active ingredient a highly safe substance having the effect of inhibiting the growth of Thermo-Acidophilic Bacilli.

### DESCRIPTION OF EMBODIMENTS

The Thermo-Acidophilic Bacilli inhibitor of the present invention contains xanthohumol as an active ingredient.

Xanthohumol is a component contained in hops, which are an edible plant, and are appropriate for food and beverages. Meanwhile, Thermo-Acidophilic Bacilli are resistant to heat and grow under acidic conditions, and thus problematically grow in food and beverages, especially in beverages. The Thermo-Acidophilic Bacilli inhibitor of the present invention is therefore preferably for beverages.

Furthermore, since the possibility for Thermo-Acidophilic Bacilli to grow at a pH of 6.5 or lower is not denied (Non-Patent Literature: Jpn. J. Food Microbiol. published by Japanese Society of Food Microbiology, 23(4), 204-212, 2006), the Thermo-Acidophilic Bacilli inhibitor of the present invention is preferably for beverages having a pH of 6.5 or lower.

Xanthohumol is a component derived from hops and can be obtained by extraction from hops using a solvent. For example, xanthohumol is extracted by immersing pellets, formed from dried hops through pulverization or the like, in an organic solvent such as an alcohol. The resulting extract is then condensed and dried, followed by separation and purification by a technique such as chromatography.

Since xanthohumol is commercially available, a commercially available product can also be used.

The present invention also relates to a method of preventing contamination of a beverage by Thermo-Acidophilic Bacilli, including adding xanthohumol to a concentration of 3.1 mass ppm or more.

Adding xanthohumol to a beverage to a concentration of 3.1 mass ppm or more can sufficiently achieve the effect of preventing the growth of Thermo-Acidophilic Bacilli.

Xanthohumol is more preferably added to a concentration of 6.2 mass ppm or more.

A larger amount of xanthohumol added is expected to produce a better effect of preventing contamination by Thermo-Acidophilic Bacilli. Yet, the method of preventing contamination of a beverage by Thermo-Acidophilic Bacilli according to the present invention preferably includes adding xanthohumol to a beverage to a concentration of 500 mass ppm or less in order to maintain the essential flavor of the beverage.

Xanthohumol is preferably added to a beverage to a concentration of 3.1 to 500 mass ppm, more preferably to 6.2 to 500 mass ppm.

Xanthohumol has unique sourness and bitterness.

Bitterness and sourness are included in the five basic tastes, namely sweetness, sourness, bitterness, saltiness, and umami, and are the sensations produced by stimulation of taste buds on the tongue. The taste and deliciousness of food or a beverage are based on various factors, including the basic tastes perceived by the taste buds as described above, pain caused by direct stimulation of skin in the mouth, temperature sensation, and smell perceived by the nose, and are therefore a variety of information perceived together.

A known method of reducing sourness or bitterness of a beverage or the like is typically adding a substance having sweetness, such as a sugar, an amino acid, or a nucleic acid. This method does not remove the sourness or bitterness itself but covers the sourness and bitterness with strong sweetness.

Sweetness is most perceivable in the body temperature (37°C) range, but unfortunately tends to be less perceivable due to the thermal stimulation in the low temperature range (5°C or lower) and the high temperature range (55°C or higher). In contrast, bitterness and sourness are highly perceivable also in the low temperature range. Sourness tends to be perceivable in the high temperature range as well. Thus, a low temperature (5°C or lower) beverage has less perceivable sweetness and more perceivable bitterness and sourness than a room temperature (25°C) beverage containing the same components as the low temperature beverage. The room temperature beverage, in contrast, has more perceivable sweetness and less perceivable bitterness and sourness since the sweetness masks the bitterness and sourness.

Solid foods and beverages are different in their forms as they are solid and liquid, respectively. Beverages, which are liquid, spread in the mouth immediately after being put in the mouth and come into contact with many of the taste buds on the tongue, tending to have a stronger taste and be more perceivable.

Accordingly, the taste and deliciousness of a food or beverage perceived by a human are influenced by the temperature and conditions of the food or beverage as well as the components in the food or beverage and the amounts thereof.

Xanthohumol produces sourness and bitterness when added in a large amount to a beverage, although Patent Literature 3 discloses that xanthohumol itself has no bitterness.

From the viewpoint of the beverage flavor, xanthohumol is more preferably added to a beverage to a concentration of 200 mass ppm or less, still more preferably to 180 mass ppm or less. A beverage according to one embodiment of the present invention preferably contains 200 mass ppm or lower, more preferably 180 mass ppm or lower, of xanthohumol.

The Thermo-Acidophilic Bacilli inhibitor and the method of preventing contamination of a beverage by Thermo-Acidophilic Bacilli according to the present invention are preferably used for beverages having a pH of 6.5 or lower. Thermo-Acidophilic Bacilli, though depending on the strains, may grow in the acidic range with a pH of 6.5 or lower, and are reported not to grow at a pH of 2.0 or lower and a pH of 6.5 or higher. Also in the neutral range with a pH of 6.5 or higher, Thermo-Acidophilic Bacilli may not die and may still cause the contamination problem even when heated under the sterile conditions in conformity with the Food Sanitation Act. In addition, the Thermo-Acidophilic Bacilli inhibitor and the method of preventing contamination of a beverage by Thermo-Acidophilic Bacilli according to the present invention are more preferably used for beverages having a pH of 4.6 or lower, more preferably for beverages having a pH of 4.0 or lower. This is because there remains a risk of microbial contamination by Thermo-Acidophilic Bacilli after the thermal sterilization in conformity with the production standards under the Food Sanitation Act where beverages having a pH lower than 4.0 need to be thermally sterilized at 65°C for 10 minutes or under equivalent or better conditions, and those having a pH of 4.0 or higher and lower than 4.6 at 85°C for 30 minutes or under equivalent or better conditions. For example, there is a report that in order to reduce the number of viable bacteria belonging to the genus *Alicyclobacillus* to 1/10, heating in the case of a sterilization temperature of 90°C needs to be run for 10 minutes or longer (Food & Packaging, 2015, Vol. 56, No. 3). This article suggests that sterilization under the conditions in conformity with the Food Sanitation Act may be insufficient to kill Thermo-Acidophilic Bacilli in soft drinks having a pH lower than 4.6.

In one embodiment, the method of preventing contamination of a beverage by Thermo-Acidophilic Bacilli according to the present invention preferably includes adjusting the pH of a beverage to 6.5 or lower.

The method of preventing contamination by Thermo-Acidophilic Bacilli according to the present invention involves an antibacterial action against Thermo-Acidophilic Bacilli that may grow in the acidic range with a pH of 6.5 or lower and that are reported not to grow at a pH of 2.0 or lower and a pH of 6.5 or higher. In the neutral range with a pH of 6.5 or higher, Thermo-Acidophilic Bacilli may not die and may still cause the contamination problem even when heated under the sterile conditions in conformity with the Food Sanitation Act.

Also, since the method of the present invention is appropriately used to prevent contamination of a beverage having a pH of 4.6 or lower by Thermo-Acidophilic Bacilli as described above, the method more preferably includes adjusting the pH of the beverage to 4.6 or lower. The method may include adjusting the pH of the beverage to lower than 4.6. This is because there remains a risk of microbial contamination by Thermo-Acidophilic Bacilli after the thermal sterilization in conformity with the production standards under the Food Sanitation Act where beverages having a pH lower than 4.0 need to be thermally sterilized at 65°C for 10 minutes or under equivalent or better conditions, and those having a pH of 4.0 or higher and lower than 4.6 at 85°C for 30 minutes or under equivalent or better conditions.

The pH of a beverage required to be the above pH value is the final pH.

Xanthohumol is converted to isoxanthohumol by heating. The heating conditions for the conversion are 80°C to 140°C for 15 minutes to 5 hours. The thermal sterilization conditions according to the production standards under the Food Sanitation Act for beverages having a pH lower than 4.0 are 65°C for 10 minutes or better conditions, where xanthohumol is still resistant to heat and are therefore not converted to isoxanthohumol. Thus, the method of preventing contamination of a beverage by Thermo-Acidophilic Bacilli according to one embodiment of the present invention more preferably includes adjusting the pH of the beverage to 4.0 or lower. The method may include adjusting the pH of the beverage to lower than 4.0.

The pH can be adjusted as needed by, for example, a known method such as adding a pH adjustor.

Herein, the Thermo-Acidophilic Bacilli are bacteria belonging to the genus Alicyclobacillus, including *Alicyclobacillus acidoterrestris* and Alicyclobacillus acidifilus. In particular, *Alicyclobacillus acidoterrestris* are preferred. Xanthohumol is effective in preventing contamination by these bacteria.

The growth pH for Thermo-Acidophilic Bacilli is usually 2 to 6, and there is a report that they do not grow at a pH of 6.5 or higher. In the neutral range (pH of 6.5 or higher), these bacteria do not grow but do not die either.

A beverage for which the Thermo-Acidophilic Bacilli inhibitor and the method of preventing contamination of a beverage by Thermo-Acidophilic Bacilli according to the present invention are used is specifically described below. The beverage is preferably a tea-based beverage, coffee beverage, alcohol beverage, non-alcoholic beer taste beverage, carbonated beverage, functional beverage, fruit and/or vegetable-based beverage, lactic beverage, soy milk beverage, or flavored water. This is because the above beverages are desired to have a pH of 6.5 or lower and an antibacterial action against Thermo-Acidophilic Bacilli. In particular, the beverage is more preferably a non-alcoholic beer taste beverage, carbonated beverage, functional beverage, or fruit and vegetable beverage which typically have a pH of 4.6 or lower because the growth possibility for Thermo-Acidophilic Bacilli in these beverages is high.

The present invention also relates to a beverage containing more than 12 mass ppm and 180 mass ppm or less of xanthohumol and having a pH of 6.5 or lower.

An acidic beverage having a pH of 6.5 or lower and containing more than 12 mass ppm of xanthohumol can achieve an even better effect of inhibiting the growth of Thermo-Acidophilic Bacilli. Moreover, an acidic beverage having a pH of 6.5 or lower and containing 180 mass ppm or less of xanthohumol can achieve a sufficient effect of inhibiting the growth of Thermo-Acidophilic Bacilli without spoiling its flavor. The pH can be measured with a commercially available pH meter.

The beverage of the present invention preferably contains 60 mass ppm or more of xanthohumol. The amount of xanthohumol can be measured by, for example, a quantitative analysis by high performance liquid chromatography (HPLC) or an LC-MS/MS system (TSQ Quantiva, Thermo Fisher Scientific Inc.). With a high performance liquid chromatograph (HPLC), the amount of xanthohumol can be measured by a method under the following conditions, for example.

### (Basic conditions)

Device: SHIMADZU LC-20AD (available from Shimadzu Corporation)
Flow rate: 1.0 mL/min
Analysis time: 25 min/sample
Column: Nomura Chemicals Develosil C30-UG-5, 4.6 mmφ × 150 mm
Column temperature: 40°C
Detector: SPD-20A
Detection wavelength: 350 nm

### (Mobile phase)

Phase A: 0.1% formic acid aqueous solution
Phase B: 0.1% formic acid-containing acetonitrile

### (Gradient conditions)

Table 1 shows gradient conditions. The percentage (%) of phase B is v/v%.

**[Table 1]**

| Analysis time (min) | Phase A | Phase B |
|---|---|---|
| 0 | 85 | 15 |
| 1.5 | 85 | 15 |
| 6 | 0 | 100 |
| 14 | 0 | 100 |
| 20 | 85 | 15 |
| 25 | 85 | 15 |

The beverage of the present invention is preferably a tea-based beverage, coffee beverage, alcohol beverage, non-alcoholic beer taste beverage, carbonated beverage, functional beverage, fruit and/or vegetable-based beverage, lactic beverage, soy milk beverage, or flavored water.

When the beverage of the present invention is a tea-based beverage, preferably, it is a black tea beverage or sugarless tea beverage. Examples of the sugarless tea beverage include green tea beverages, oolong tea beverages, barley tea beverages, brown rice tea beverages, adlay tea beverages, and sugarless black tea beverages.

When the beverage of the present invention is a coffee beverage, preferably, it is a packaged coffee beverage or liquid coffee.

Examples of the alcohol beverage include beer, beer-based beverages, and alcohol beverages other than the beer and beer-based beverages.

When the beverage of the present invention is a beer-based beverage, preferably, it is low-malt beer or a beer-like beverage.

When the beverage of the present invention is an alcohol beverage other than the beer and beer-based beverages, preferably, it is *shochu,* a *shochu* highball, liqueur, cocktail, spirit, or whisky.

The term "non-alcoholic beer taste beverage" as used herein refers to carbonated beverages with beer-like flavors of non-fermented, non-alcohol type, which are substantially free of alcohols. Here, the non-alcoholic beer taste beverage does not exclude beverages containing a very trace amount (undetectable degree) of alcohol.

When the beverage of the present invention is a carbonated beverage, preferably, it is a cola-flavored beverage, clear carbonated beverage, ginger ale, fruit juice-based carbonated beverage, milk-containing carbonated beverage, or sugarless carbonated beverage.

When the beverage of the present invention is a functional beverage, preferably, it is a sports drink, energy drink, health-supporting beverage, or jelly drink pouch.

When the beverage of the present invention is a fruit and/or vegetable-based beverage, preferably, it is a 100% fruit juice, fruit-containing beverage, soft drink with a low fruit juice content, pulp-containing fruit juice, or pulp-containing beverage.

When the beverage of the present invention is a lactic beverage, preferably, it is milk, a yogurt drink, lactic acid bacteria beverage, or milk-containing soft drink.

When the beverage of the present invention is a soy milk beverage, preferably, it is soy milk or a soybean beverage.

The beverage of the present invention is preferably a non-alcoholic beer taste beverage, carbonated beverage, functional beverage, or fruit and/or vegetable-based beverage which have a pH of 4.6 or lower, more preferably a functional beverage or fruit and/or vegetable-based beverage. Here, the pH of the beverage is 4.6 or lower and may be lower than 4.6. The pH of the beverage is preferably 3.0 or higher.

This is because there remains a risk of microbial contamination by Thermo-Acidophilic Bacilli after the thermal sterilization in conformity with the production standards under the Food Sanitation Act where beverages having a pH lower than 4.0 need to be thermally sterilized at 65°C for 10 minutes or under equivalent or better conditions, and those having a pH of 4.0 or higher and lower than 4.6 at 85°C for 30 minutes or under equivalent or better conditions.

Xanthohumol is converted to isoxanthohumol by heating. The heating conditions for the conversion are 80°C to 140°C for 15 minutes to 5 hours. The thermal sterilization conditions according to the production standards under the Food Sanitation Act for beverages having a pH lower than 4.0 are 65°C for 10 minutes or better conditions, where xanthohumol is still resistant to heat and are therefore not converted to isoxanthohumol. Thus, in one embodiment, the beverage preferably has a pH of 4.0 or lower or lower than 4.0.

In a preferred embodiment, the beverage of the present invention has an antibacterial action against Thermo-Acidophilic Bacilli.

The beverage of the present invention and a beverage for which the Thermo-Acidophilic Bacilli inhibitor and the method of preventing contamination by Thermo-Acidophilic Bacilli according to the present invention are used are each preferably a beverage to be served in the low temperature range of 5°C or lower (e.g., 4°C to 5°C) and/or the high temperature range of 55°C or higher (e.g., 55°C to 95°C). This is because although the beverage of the present invention contains xanthohumol having an antibacterial action against Thermo-Acidophilic Bacilli, xanthohumol, contained at a certain concentration that does not spoil the flavor of the beverage, does not spoil the flavor of the beverage in the low temperature range where bitterness and sourness are easily perceived and in the high temperature range where sourness is easily perceived.

The form of the beverage is not particularly limited. Examples include packaged beverages. Packages for the packaged beverages are not particularly limited. Packages in any form and of any material may be used. For example, any of the following commonly used packages can be used: metal packages such as aluminum cans and steel cans; resin containers such as PET bottles; paper containers such as drink cartons; glass containers such as glass bottles; and wooden containers such as barrels. Any of these packages is filled with the beverage and sealed, whereby a packaged beverage can be obtained.

The beverage can be prepared, for example, by adding xanthohumol to a material used in the beverage production (e.g., a food raw material or food additive).

### EXAMPLES

The following provides examples that more specifically describe the present invention. The present invention is not limited to these examples.

### <Preparation Example 1>

### Preparation of xanthohumol

Xanthohumol was isolated and purified from a hop extract (Asama Chemical. Co., Ltd.) by the following method. Specifically, using a hop extract as a raw material, xanthohumol was purified by normal-phase column chromatography, reverse phase column chromatography, and preparative HPLC, and the purity was determined to be 95% or higher by HPLC analysis. For HPLC analysis, a Develosil C30-UG-5 column (Nomura Chemical Co., Ltd.) was used, and the detector wavelength to measure UV absorption was 350 nm. The obtained xanthohumol was used as a standard sample (having a purity of 95% or higher) in the following experiments.

### <Example 1>

### Evaluation of antibacterial action

Thermo-Acidophilic Bacilli (*Alicyclobacillus acidoterrestris* ATCC 49025) were pre-incubated as test bacteria (conditions: YSG broth, 50±1°C, two days, aerobic conditions), so that a test bacterial solution was obtained.

A 10 mg/mL xanthohumol solution and serial two-fold dilutions thereof were prepared using 99.5% ethanol. Each of the solution and dilutions was added to a medium (YSG agar medium) whose temperature is maintained at 50±1°C after being sterilized and dissolved at a ratio of 1/99 (volume of xanthohumol solution/volume of medium solution) and mixed sufficiently. The resulting mixtures were each dispensed into a petri dish and solidified, whereby agar plate media were produced.

Each of the agar plate media was smeared with the pre-incubated test bacterial solution, followed by incubation at 50±1°C for five days under aerobic conditions. The lowest concentration where the growth of the bacteria was prevented was taken as the minimum inhibitory concentration (MIC). Table 2 below shows the results.

**[Table 2]**

| Test bacteria | Test substance | MIC (µg/mL) |
|---|---|---|
| Thermo-Acidophilic Bacilli (*Alicyclobacillus acidoterrestris* ATCC 49025) | Xanthohumol | 3.13 |

Xanthohumol was found to inhibit the growth of Thermo-Acidophilic Bacilli (*Alicyclobacillus acidoterrestris*)*.*

### INDUSTRIAL APPLICABILITY

The Thermo-Acidophilic Bacilli inhibitor, the method of preventing contamination of a beverage by Thermo-Acidophilic Bacilli, and the beverage according to the present invention are useful in the food and beverage field.

## Claims

1. A Thermo-Acidophilic Bacilli inhibitor comprising
xanthohumol as an active ingredient.

2. The Thermo-Acidophilic Bacilli inhibitor according to claim 1,
which is for beverages use.

3. The Thermo-Acidophilic Bacilli inhibitor according to claim 1 or 2,
wherein the Thermo-Acidophilic Bacilli are *Alicyclobacillus acidoterrestris.*

4. A method of preventing contamination of a beverage by Thermo-Acidophilic Bacilli, comprising
adding xanthohumol to a concentration of 3.1 mass ppm or more.

5. The method of preventing contamination by Thermo-Acidophilic Bacilli according to claim 4,
wherein xanthohumol is added to a concentration of 6.2 mass ppm or more.

6. The method of preventing contamination by Thermo-Acidophilic Bacilli according to claim 4 or 5,
further comprising adjusting a pH of the beverage to 6.5 or lower.

7. The method of preventing contamination by Thermo-Acidophilic Bacilli according to any one of claims 4 to 6,
wherein the Thermo-Acidophilic Bacilli are *Alicyclobacillus acidoterrestris.*

8. A beverage comprising more than 12 mass ppm and 180 mass ppm or less of xanthohumol and having a pH of 6.5 or lower.

9. The beverage according to claim 8,
which comprises 60 mass ppm or more of xanthohumol.

10. The beverage according to claim 8 or 9,
which is a tea-based beverage, coffee beverage, alcohol beverage, non-alcoholic beer taste beverage, carbonated beverage, functional beverage, fruit and/or vegetable-based beverage, lactic beverage, soy milk beverage, or flavored water.

11. The beverage according to any one of claims 8 to 10,
which is a non-alcoholic beer taste beverage, functional beverage, carbonated beverage, or fruit and/or vegetable-based beverage and has a pH of 4.6 or lower.
